# EUROPEAN PATENT SPECIFICATION

(11) **EP 3 470 835 B1**
(45) Date of publication and mention of the grant of the patent: **22.03.2023**
(21) Application number: 17196426.5
(22) Date of filing: 13.10.2017
(51) Int. Cl.: G01N 33/00, G01N 1/24, G01N 33/22, G01N 33/497

(54) **FLUID SENSING DEVICE FOR A PORTABLE ELECTRONIC DEVICE**
FLUIDMESSVORRICHTUNG FÜR EINE TRAGBARE ELEKTRONISCHE VORRICHTUNG
DISPOSITIF DE DÉTECTION DE FLUIDE POUR DISPOSITIF ÉLECTRONIQUE PORTABLE

(43) Date of publication of application: 17.04.2019
(73) Proprietor: BoydSense, Inc., South San Francisco, CA 94080 (US)
(72) Inventor: THUILLIER, Bruno, San Bruno, CA 94066 (US)
(74) Representative: Grünecker Patent- und Rechtsanwälte PartG mbB

(56) References cited:
- US-A1- 2014 134 053
- US-A1- 2016 103 104
- US-A1- 2017 184 556
- JING LIU ET AL: "A pressure programmable gas chromatography microsystem utilizing motionless Knudsen pump, fiber-integrated optical detector, and silicon micromachined separation column", 2011 16TH INTERNATIONAL SOLID-STATE SENSORS, ACTUATORS AND MICROSYSTEMS CONFERENCE (TRANSDUCERS 2011) ; BEIJING, CHINA; 5 - 9 JUNE 2011, IEEE, PISCATAWAY, NJ, 5 June 2011 (2011-06-05), pages 803-806, XP031910632, DOI: 10.1109/TRANSDUCERS.2011.5969291 ISBN: 978-1-4577-0157-3
- GUPTA NAVEEN ET AL: "Thermal transpiration in zeolites: A mechanism for motionless gas pumps", APPLIED PHYSICS LETTERS, A I P PUBLISHING LLC, US, vol. 93, no. 19, 14 November 2008 (2008-11-14), pages 193511-193511, XP012112373, ISSN: 0003-6951, DOI: 10.1063/1.3025304
- GRZEBYK TOMASZ: "MEMS Vacuum Pumps", JOURNAL OF MICROELECTROMECHANICAL SYSTEMS, IEEE SERVICE CENTER, US, vol. 26, no. 4, August 2017 (2017-08), pages 705-717, XP011657906, ISSN: 1057-7157, DOI: 10.1109/JMEMS.2017.2676820 [retrieved on 2017-07-31]

## Description

The invention relates to a portable telecommunication device comprising at least a fluid sensing device equipped with a fluid sensor.

Small size fluid sensors, such as small size metal oxide (MOX) sensors or carbon nanotube (CNT) sensors are known and may be used, for example, to monitor indoor air quality, to estimate the blood alcohol content from a user's exhalation or for military purposes with wearable toxic-gas detectors.

Such fluid sensors may be directly integrated into a portable electronic device. In this case, the fluid sensor is accessible though openings in the housing, like the openings for a microphone as described in the prior art document EP3187865 A1; or via other openings, e.g. a speaker of the portable electronic device. As an alternative to built-in fluid sensors, plug-in fluid sensor modules may be connected to portable electronic devices.

However, depending on the sensing device's location, adding fluid analysis capability to smartphones and wearables may require to actively feed the fluid towards, through and/or out of the sensing device. Fluid moving means are known in the art and comprise mechanical moving parts, such as mechanical pumps or fans, that are not suitable for mobile devices like cell phones, smartphones, smartwatches, portable computers or tablet computers because of their high energy consumption, their bulkiness and the emitted noise level. Fluid moving means for miniature gas sensors for portable electronic devices are for example Knudsen pumps. Documents disclosing such Knudson pumps are, for example
US 2017/184556 A1,
US 2016/103104 A1,
JING LIU ET AL: "A pressure programmable gas chromatography microsystem utilizing motionless Knudsen pump, fiber-integrated optical detector, and silicon micromachined separation column", 2011 16TH INTERNATIONAL SOLID-STATE SENSORS, ACTUATORS AND MICROSYSTEMS CONFERENCE (TRANSDUCERS 2011); BEIJING, CHINA; 5 - 9 JUNE 2011, IEEE, pages 803-806, XP031910632, DOI: 10.1109/TRANSDUCERS.2011.5969291,
GUPTA NAVEEN ET AL: "Thermal transpiration in zeolites: A mechanism for motionless gas pumps", APPLIED PHYSICS LETTERS, A I P PUBLISHING LLC, US, vol. 93, no. 19, 14 November 2008, pages 193511-193511, XP012112373, ISSN: 0003-6951, DOl: 10.1063/1.3025304,
GRZEBYK TOMASZ: "MEMS Vacuum Pumps", JOURNAL OF MICROELECTROMECHANICAL SYSTEMS, IEEE SERVICE CENTER, US, vol. 26, no. 4, August 2017), pages 705-717, XP011657906, ISSN: 1057-7157, DOl: 10.1109/JMEMS.2017.2676820
Other fluid moving means for use in gas sensing applications in mobile phones are known from US 2014/134053 A1.

It is therefore an object of the present invention to provide a fluid sensing device for a portable electronic device that overcomes the problems mentioned above. In particular, it is the object of the present invention to provide a fluid sensing device that consumes less energy and/or less volume demanding than a mechanical gas moving device and/or operates more silently. It is also an object to provide a portable electronic device comprising such a fluid sensing device as well as a method for moving a fluid also overcoming the above mentioned problems.

The object of the invention is achieved with a portable telecommunication device comprising at least a fluid sensing device according to claim 1. The fluid sensing device comprises a fluid sensing means configured to analyze a sampling volume; and a fluid moving means comprising a motionless fluid pumping means using thermal transpiration for moving the sampling volume. The object of the invention is thus achieved with a motionless gas pump operating by thermal transpiration, also called a Knudsen pump. A Knudsen pump comprises narrow channels and/or a porous media under temperature gradient. By allowing the fluid to flow from the cold end to the hot end, i.e. by thermal transpiration, the Knudsen pump generates a flow of a fluid with no moving parts. Since Knudsen pumps do not comprise any moving parts, the inventive device operates more silently. Furthermore, the size of the fluid moving means is reduced, as Knudsen pumps can be more compact than mechanical pumps, and thus are suitable for portable electronic device. In addition, unlike mechanical gas pumps, Knudsen pumps only require heat, as an energy source. As a result, the inventive fluid sensing device comprising a motionless gas pump operating by thermal transpiration consumes less energy, takes less place and operates more silently compared to prior art fluid moving means. The Knudsen pump can be used to move the fluid towards the sensing means and/or to move it away from the sensing means after analysis.

The present fluid sensing device for a portable electronic device can be further improved according to various advantageous embodiments.

According to one embodiment of the invention, the fluid sensing device can operate with a gas as fluid. Hence, the fluid sensing device could be used, for example, to probe a user's breath for the purpose of monitoring blood alcohol content or some health indicators. In another example, the fluid sensing device could also be used to monitor the environment, i.e. ambient air, or to detect toxic gas.

According to one embodiment of the invention, the motionless gas pumping means can be powered by a heat source. Furthermore, according to another embodiment of the invention, the heat source of the motionless fluid pumping means can be based on Joule effect and/or solar energy. As a result, unlike mechanical gas pumps that rely on AC driving voltages; providing a heat source by means of DC voltages may be sufficient to power motionless fluid pumping means using thermal transpiration, which consequently, operate with simple electronic control circuitry and low energy consumption.

According to one embodiment of the invention, the fluid sensing device for a portable electronic device can further comprise a battery and/or electronic components used as the heat source of the motionless fluid pumping means. In particular, it is the battery and/or electronic components that can be used for other functionalities of the portable electronic device that can be used as the heat source for the Knudsen pump. This kind of heat source is already available in portable electronic devices so that no extra energy source needs to be added. The Knudsen pump takes thus advantage of unused wasted energy. Therefore, the number of components can be kept low, and consequently the weight of the fluid sensing device remains low.

According to one embodiment of the invention, the fluid moving means can be capable of adjusting the flow rate and/or the temperature of the sampling volume. Indeed, the fluid of the sampling volume can be processed and preheated by the fluid moving means, to prepare the sampling volume such that it can be analyzed by the fluid sensing means. Adjusting the flow rate and/or the temperature of the gas sampling volume can also exercised a catalytic effect on the fluid sensing means, in particular on metal oxide based fluid sensing means.

According to one embodiment of the invention, the fluid sensing means can be sensitive to at least one type of molecule. For example, the fluid sensing means can be sensitive to carbon dioxide (CO₂) in ambient air or in the exhaled air of a user.

According to one embodiment of the invention, the fluid sensing means comprises a metal oxide (MOX) sensor and/or a carbon nanotube (CNT) sensor. MOX sensors and CNT sensors can provide reliable fluid analysis. Moreover, the CNT sensor has, in particular, low energy consumption.

According to one embodiment of the invention, the fluid sensing device can further comprise a reverse thermal transpiration-based fluid pumping means configured for ejecting the sampling volume. Therefore, the fluid sensing device can expel the gas out of the fluid sensing device, once the gas has been analyzed by the fluid sensing means.

According to one embodiment of the invention, the motionless fluid pumping means can further comprise a reversible motionless fluid pumping means using thermal transpiration for moving the sampling volume with a bidirectional flow. Hence, the number of components can be reduced, as the same fluid pumping means is used to move the fluid towards the device as well as to eject it.

According to the invention the fluid sensing means is positioned within the same opening of the portable device's housing as at least one other functional unit. Thus, fluid analyzing capability can be provided without having to provide one additional hole in the device's housing.

According to the invention, the fluid sensing means is positioned further inside the housing than the at least one other functional unit. By using the motionless fluid moving means the design liberty for placing the fluid sensing device is improved.

The motionless fluid pumping means using thermal transpiration may adjust the flow rate and/or the temperature of the sampling volume. As a consequence, the gas of the sampling volume can be processed and preheated by the motionless fluid pumping means, which thus, at the same time prepares the sampling volume so that it can be analyzed in the fluid sensing means. Adjusting the flow rate and/or the temperature of the gas sampling volume can also exercise a catalytic effect on the fluid sensing means.

Additional features and advantages of the present invention will be described with reference to the drawings. In the description, reference is made to the accompanying figures that are meant to illustrate preferred embodiments of the invention. It is understood that such embodiments do not represent the full scope of the invention.
Figure 1 illustrates a schematically, perspective view of a portable telecommunication device comprising a fluid sensing device according to a first embodiment of the invention;
Figure 2 illustrates a block diagram with the components of the fluid sensing device according to the first embodiment;
Figure 3 illustrates a block diagram with components of a fluid sensing device according to a second embodiment;
Figure 4 illustrates a block diagram with components of a fluid sensing device according to a third embodiment;
Figure 5 illustrates a flowchart with the steps of a method for moving a fluid in a portable electronic device according to a fourth embodiment of the invention;
Figure 6 illustrates a chart with the steps of a method for moving a fluid within a portable electronic device according to a fifth embodiment of the invention;
Figure 7 illustrates a block diagram with the components of the fluid sensing device according to the sixth embodiment of the invention.

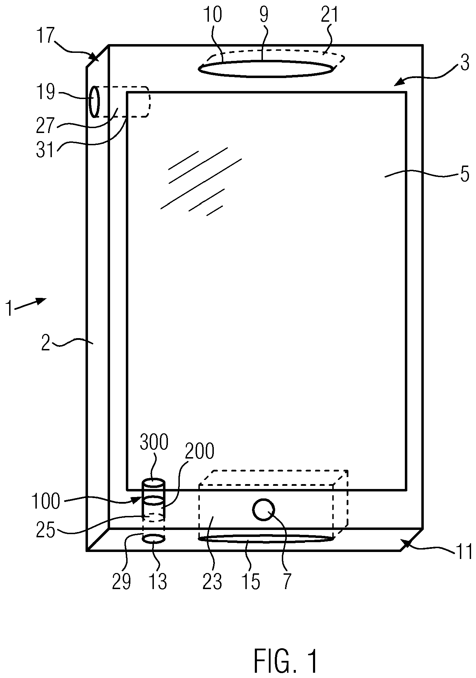

The portable electronic device 1 illustrated in figure 1 represents a portable telecommunication device according to the invention, such as a mobile phone. The portable electronic device 1 can also be a smartphone, a tablet, a laptop, a personal electronic assistant, a tracking device, an electronic wearable or the like.

The portable electronic device 1 comprises a housing 2 which in this embodiment has a rectangular shape. One of the two main sides of the portable electronic device 1 , i.e. the front side 3, is provided with a display device 5, such as a screen, and interfacing elements like a button 7, allowing the user to interact with the portable electronic device 1.

The portable electronic device may comprise one or more openings. The front side 3 is further provided with an opening 9 for a loudspeaker. The opening 9 is protected from dirt, like dust particles, by a grid 10. The housing 2 may comprise further openings 13, 15, e.g. on the lower side 11 of the display device 5. Also, the side-wall 17 of the housing 2 may be provided with another opening 19. A microphone, a loudspeaker, a headphone jack hole, a charger hole and/or the like can be provided in openings like the openings 13 and/or 15 and/or 19.

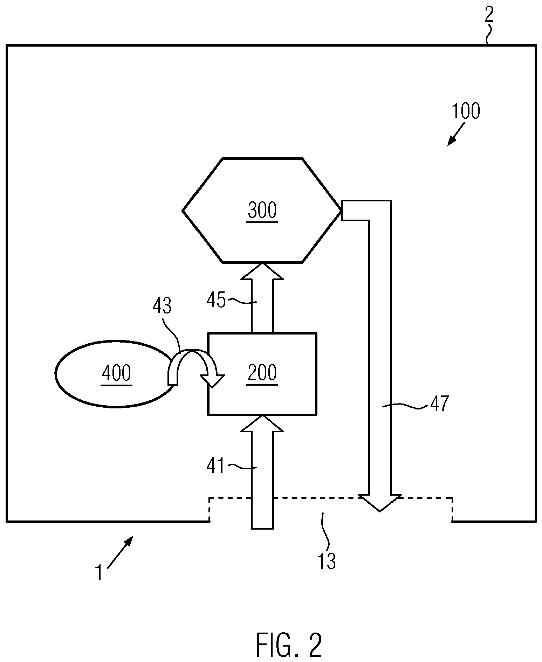

The dotted lines on figure 1 represent inner portions 21, 23, 25, 27 having various possible shapes inside the openings 9, 13, 15, 19 of the housing 2 of the portable electronic device 1. In the tubular-shaped duct 29 of the opening 13, a fluid sensing device 100 according to a first embodiment of the invention, is mounted. The fluid sensing device 100 comprises a fluid moving means 200 for moving a sampling volume towards a fluid sensing means 300 for analyzing the sampling volume. The fluid sensing device 100 will be described in more detail in figure 2.

The fluid sensing means can be a metal oxide (MOX) sensor and/or a carbon nanotube (CNT) sensor. The fluid sensing means 300 of the fluid sensing device 100 can also comprise gold nanoparticles, a silicon nanowire, a quartz crystal microbalance, a colorimetric sensor and/or a conductive polymer; which represent other low energy consumption sensing means.

The fluid moving means 200 is in a fluid connection with the opening 13 of the tubular-shaped duct 29.

In this embodiment, the opening 13 receiving the fluid sensing device 100 can be located near the fluid to analyze, e.g. located near the mouth of a user for the purpose of analyzing the user's exhaled air while the user talks on the portable electronic device 1. In other variants the fluid sensing device 100 could be positioned elsewhere like in the inner portions 21, 23 or 27 of the other openings 9, 15 or 19, depending on the available space inside the housing 2.

According to the invention the fluid sensing device 100 is placed inside one of the openings 9, 13, 15 or 19 together with one or more other functional features, like the microphone, the speaker or alike. According to the invention, due to the use of the fluid moving means 200, the fluid sensing device 100 is positioned further inside the housing 2 than the other functional feature.

Figure 2 illustrates a block diagram of the fluid sensing device 100 according to the first embodiment of the present invention, as illustrated in Figure 1. Elements with the same reference numeral already used in figure 1 will not be described in detail again but reference is made to their description above.

Figure 2 illustrates the motionless fluid pumping means 200 powered by a heat source 400 and the fluid sensing means 300.

The motionless fluid pumping means 200 generates a gas flow based on thermal transpiration using, e.g. narrow channels and/or a porous media which allowing thermal transpiration to occur to generate a flow of a fluid entering the motionless fluid pumping means 200. With a temperature gradient applied along the narrow channel, which is not represented in figure 2, the fluid flows from the cold end to the hot end. As the Knudsen pump 200 does not comprise moving parts, it operates more silently than mechanical gas pumps, and thus is well-adapted for use in portable electronic device.

To create the temperature gradient, the motionless fluid pumping means 200 uses a heat source 400 of the portable electronic device 1.The motionless fluid pumping means 200 operates with simple electronic control circuitry and low energy consumption.

The heat source 400, represented in figure 2, can be a battery 400 enclosed in the housing 2 of the portable electronic device 1. The battery 400 can, for example, be a rechargeable battery used to run the various functionalities of the portable electronic device 1. By placing the heat source 400 in the vicinity of the motionless fluid pumping means 200 one can take advantage of the heat 43 created by the battery 400. Thus, the usually wasted thermal energy can be used according to the invention to run the motionless fluid pumping means 200.

The heat source 400 can also comprise any of the other components of the portable electronic device 1 that heat up during use or the heat a solar energy conversion means.

The fluid sensing device 100 according to the first embodiment functions as follows:
a sampling volume 41 of a fluid to be analyzed, e.g. the breath of a user or the ambient air, is drawn into the opening 13 in the housing 2 by the motionless fluid pumping means 200 of the fluid sensing device 100. Due to the temperature gradient provided by the heat source 400, the sampling volume is then moved towards the fluid sensing means 300, as illustrated by reference numeral 45.

The parameters of the sampling volume 41, like temperature, pressure and/or flow rate, can be controlled when passing through the motionless fluid pumping means 200.

It becomes thus possible to prepare the sampling volume 41 such that its temperature and/or flow rate is adjusted in accordance with the operating parameters imposed by the fluid sensing means 300. Hence, additional preheating operations required inside the fluid sensing means 300 can be reduced or even eliminated. By adjusting the flow rate and/or the temperature of the gas sampling volume 41, it becomes, for example, possible to enable a catalytic effect needed for sensing the sampling volume 45 in the fluid sensing means 300.

As illustrated in figure 2, the sampling volume 45 is analyzed by the fluid sensing means 300 which is sensitive to at least one type of molecule. The fluid sensing means 300 can be configured to analyze ambient air, e.g. for checking air quality, and/or to analyze a user's exhalation, e.g. for measuring blood alcohol content.

The sampling volume then leaves the fluid sensing means 300 via opening 13 as illustrated by reference numeral 47.

Instead of taking different paths as illustrated in figure 2 but leaving via the same opening 13, the input sampling volume 41 and the output sampling volume 47 can move through different openings and paths of the housing 2 (not shown).

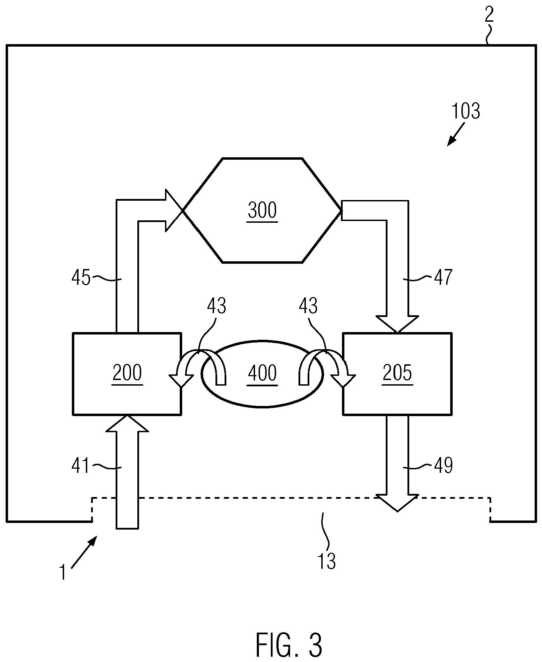

The figure 3 illustrates a block diagram of a fluid sensing device 103 according to a second embodiment of the present invention. Elements with the same reference numeral already used in figures 1 and 2 will not be described in detail again but reference is made to their description above.

As already described with respect to the first embodiment and illustrated figure 2, an input sampling volume 41 is moved towards the fluid sensing means using the motionless fluid pumping means 200. In this embodiment, however, an additional reverse thermal transpiration-based fluid pumping means 205 is used to move the output sampling volume 47 out of the fluid sensing means 300 towards the opening 13 or any other opening in the portable electronic device 1 (as indicated by the reference numeral 49).

The reverse thermal transpiration-based fluid pumping means 205 operates in the opposite flow direction compared to the motionless fluid pumping means 200. The motionless fluid pumping means 200 and the reverse motionless fluid pumping means 205 can have a common heat source 400, like illustrated in figure 3 to provide the necessary heating 43 to each one of the motionless fluid pumping means 200, 205.

In a variant, not illustrated in figure 3, each one of the motionless fluid pumping means 200, 205 has its own heat source.

Depending on the process parameters, the ejected sampling volume 47 is cooled down in the motionless fluid pumping means 205 before leaving the portable electronic device 1.

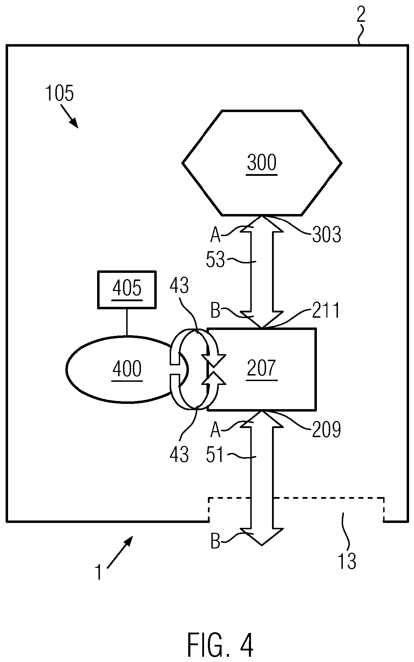

Figure 4 illustrates a block diagram of a fluid sensing device 105 according to a third embodiment of the present invention. Elements with the same reference numeral already used in figures 1, 2 and 3 will not be described in detail again but reference is made to their description above.

The fluid sensing means 105 represented in figure 4 comprises a reversible motionless fluid pumping means 207 configured to operate in two opposite directions.

In configuration A, the reversible motionless fluid pumping means 207 forces the fluid from the opening 13 towards the fluid sensing means 300. In this configuration, the cold side of the motionless fluid pumping means 207 is located in the area 209, facing the opening 13 in the housing 2, whereas the hot side of the pumping means 207 is located in the area 211, facing the fluid sensing means 300.

In configuration B, the reversible motionless fluid pumping means 207, forces the fluid from the fluid sensing means 300 towards the opening 13 of the housing 2. The cold side of the pumping means 207 is then located in the area 211 facing the fluid sensing means 300, whereas the hot side of the pumping means 207 is located in the area 209, facing the opening 13.

The reversible motionless fluid pumping means 207 comprises a thermoelectric material to be able to change the direction of the pumping by reversing the electrical current passing through the thermoelectric material, and thus the hot side and the cold side of the reversible motionless fluid pumping means 207 can be exchanged.

In use, the fluid sampling volume 51 is dragged into the housing 2 via the opening 13 by the reversible motionless fluid pumping means 207 in configuration A and pushed (indicated by reference numeral 53) towards the fluid sensing means 300, where it is analyzed.

Once the sampling volume 53 has been analyzed, a controlling means 405 changes the operational state of the reversible motionless fluid pumping means 207 to operate in configuration B. Hence, the sampling volume 53 is moved away from the fluid sensing means 300 towards the opening 13 of the fluid sensing means 105. The sampling volume 51 is then expelled from the housing 2 of the portable electronic device 1.

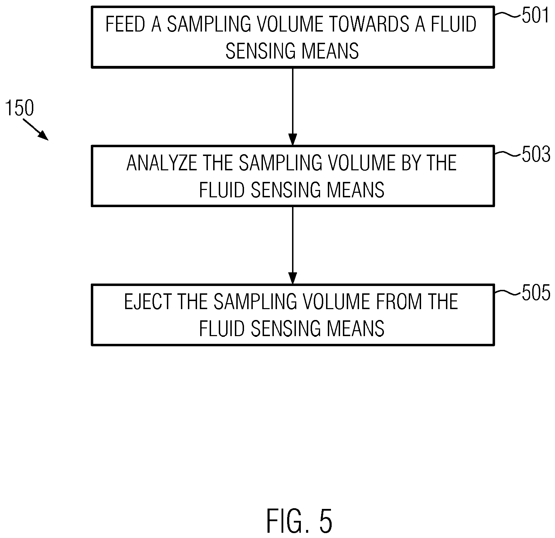

Figure 5 illustrates a flowchart 150 illustrating the steps in a method for moving a fluid in a portable electronic device according to the fourth embodiment of the invention. Elements with the same reference numeral already used in the embodiments 1 to 3 illustrated in figures 1 to 4 will not be described in detail again but reference is made to their description above.

In the first step 501 of the method for moving a fluid in a portable device 1 a sampling volume is fed towards the fluid sensing means 300 using a motionless fluid pumping means 200, 207 using thermal transpiration like described above with respect to one of embodiments 1 to 3. While moving the sampling volume towards the fluid sensing means 300, the fluid parameters, such as fluid temperature, flow rate and/or pressure can be adjusted.

In the next step 503 the sampling volume is analyzed by the fluid sensing means 300 as described above.

In step 505 the sampling volume is ejected from the fluid sensing means 300 using the motionless fluid pumping means 200, 205, 207.

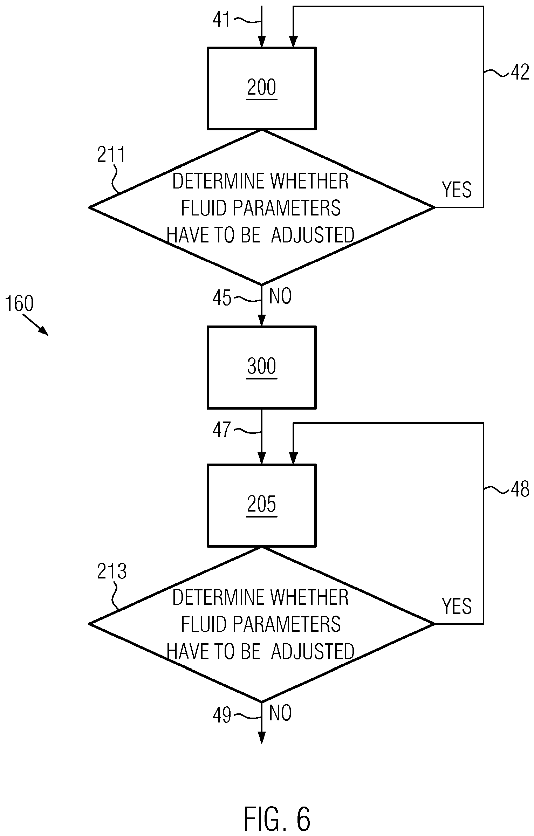

In a variant, fluid parameters, such as fluid temperature, flow rate and/or pressure can be adjusted by the motionless fluid pumping means 200, 205, 207 prior to leaving the opening 9, 13, 15 or 19 of the housing 2 of the portable electronic device 1. As a consequence, the fluid parameters can be controlled before to be ejected and thus, before that the fluid may come into contact with user's skin, hair or any material near the openings 9, 13, 15, 19 of the housing 2. Figure 6 illustrates a chart 160 with processing steps in accordance with the method for moving a fluid within a portable electronic device comprising a fluid sensing device according to a fifth embodiment of the invention. Elements with the same reference numeral already described in embodiments 1 to 4 and illustrated figures 1 to 5 will not be described in detail again but reference is made to their description above.

The sampling fluid to be analyzed, illustrated by reference numeral 41, is moved towards the fluid sensing means 300 by the motionless fluid pumping means 200.

Using the motionless fluid pumping means 200, the fluid parameters, such as the fluid flow rate, the fluid temperature and/or the pressure can be adapted. Indeed, the parameters of the sampling volume 41 are adjusted in accordance with the operating parameters of the fluid sensing means 300 and/or in accordance with a desired catalytic effect.

At step 211, it is therefore determined whether the fluid parameters of the sampling volume 41 have to be adjusted or are within the range needed by the fluid sensing means 300.

If the parameters of the sampling volume 41 have to be adjusted, the sampling volume 42 is returned to the motionless fluid pumping means 200.

If the parameters of the sampling volume 41 do not have to be further adjusted, the sampling volume 45 can thus be fed into the fluid sensing means 300.

The sampling volume 45 is then analyzed by the fluid sensing means 300.

Once the sampling volume 45 is analyzed by means of the fluid sensing means 300, the sampled volume 47 is ejected from the portable electronic device 1. In this embodiment, the sampling volume 45 is ejected by means of the reverse thermal transpiration-based fluid pumping means 205 of the second embodiment. The reverse motionless fluid pumping means 205 operates in the opposite flow direction of the motionless fluid pumping means 200. As an alternative, the reversible motionless fluid pumping means 207 of the third embodiment may also be used.

At step 213, the portable electronic device 1 determines whether the fluid parameters of the sampled volume 47 have to be adjusted before ejection.

If the parameters of the sampling volume 47 do not have to be adjusted, the sampling volume 49 is expelled out of the portable electronic device 1 via the reverse motionless fluid pumping means 205.

If the parameters of the sampling volume 47 have to be adjusted, the sampling volume 48 is returned to the motionless fluid pumping means 205, e.g. as they are too hot which might represent a risk of injury to the user of the portable electronic device 1.

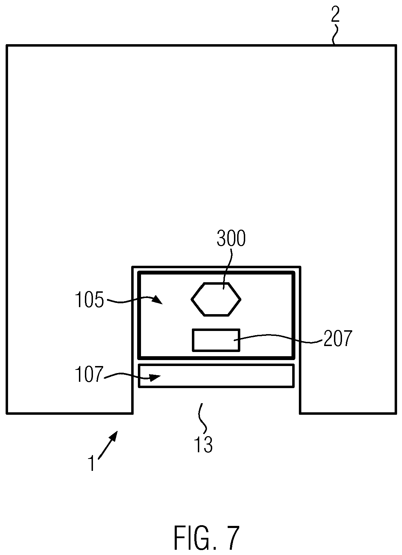

Figure 7 illustrates a block diagram of a fluid sensing device 107 according to a sixth embodiment of the invention. Elements with the same reference numeral already described in embodiments 1 to 5 and illustrated in figures 1 to 6 will not be described in detail again but reference is made to their description above.

Figure 7 illustrates the fluid sensing device 105 comprising the reversible motionless fluid pumping means 207 and the fluid sensing means 300 according to the third embodiment, as described in figure 4.

According to the sixth embodiment of the invention, the fluid sensing device 105 is placed inside the opening 13 together with another functional unit 107, e.g. a microphone. In particular, due to the use of the reversible fluid moving means 207, the fluid sensing device 105 is positioned further inside the housing 2 than the microphone 107, in particular behind the functional feature 107 of the portable electronic device 1.

As a consequence, no additional hole is necessary in the device's housing 2 and the fluid sensing device 105 can be positioned anywhere inside the housing where sufficient space is available.

The fluid sensing device 100, 103, 105 can, e.g., be positioned on an existing structure inside the portable electronic device 1, like on the printed circuit board PCB.

The various individual features of the embodiments 1 to 6 can be independently combined to create further variants according to the invention.

## Claims

1. A portable telecommunication device comprising at least a fluid sensing device (100, 103,105);
the fluid sensing device (100, 103,105) comprising:
a fluid sensing means (300) configured to analyze a sampling volume (41, 45, 53); and
a fluid moving means comprising a motionless fluid pumping means (200) using thermal transpiration for moving the sampling volume (41, 45, 47, 49, 51, 53);
wherein the fluid sensing device (100, 103, 105) is positioned within the same opening (13) of the portable device's (1) housing (2) as at least one other functional unit (107) and further inside said housing (2) than the at least one other functional unit (107).

2. The portable electronic device according to claim 1, wherein the fluid is a gas.

3. The portable electronic device according to claim 1 or 2, wherein the motionless fluid pumping means (200) is powered by a heat source (400).

4. The portable electronic device according to claim 3, wherein the heat source (400) of the motionless fluid pumping means (200) is based on Joule effect and/or solar energy.

5. The portable electronic device according to one of claims 1 to 4, wherein the fluid sensing means (300) is sensitive to at least one type of molecule.

6. The portable electronic device according to one of claims 1 to 5, wherein the fluid sensing means (300) comprises a metal oxide (MOX) sensor and/or a carbon nanotube (CNT) sensor.

7. The portable electronic device according to one of claims 1 to 6, further comprising a reverse thermal transpiration-based fluid pumping means (205) configured for ejecting the sampling volume (47, 49).

8. The portable electronic device according to one of claims 1 to 7, wherein the motionless fluid pumping means (200) comprises a reversible motionless fluid pumping means (207) using thermal transpiration for moving the sampling volume (51, 53) with a bidirectional flow.

## Patentansprüche

1. Tragbare Telekommunikationsvorrichtung, die wenigstens eine Fluid-Messvorichtung (100, 103, 105) umfasst;
wobei die Fluid-Messvorichtung (100, 103, 105) umfasst:
eine Fluid-Messeinrichtung (300), die zum Analysieren eines Probenentnahme-Volumens (41, 45, 53) ausgeführt ist;
sowie
eine Fluid-Bewegungseinrichtung, die eine bewegungslose Fluid-Pumpeinrichtung (200) umfasst, die thermische Transpiration zum Bewegen des Probenentnahme-Volumens (41, 45, 47, 49, 51, 53) nutzt;
wobei die Fluid-Messvorichtung (100, 103, 105) im Inneren der gleichen Öffnung (13) des Gehäuses (2) der tragbaren Vorrichtung (1) wie wenigstens eine andere Funktionseinheit (107) sowie weiter im Inneren des Gehäuses (2) angeordnet ist als die wenigstens eine andere Funktionseinheit (107).

2. Tragbare elektronische Vorrichtung nach Anspruch 1, wobei das Fluid ein Gas ist.

3. Tragbare elektronische Vorrichtung nach Anspruch 1 oder 2, wobei die bewegungslose Fluid-Pumpeinrichtung (200) von einer Wärmequelle (400) angetrieben wird.

4. Tragbare elektronische Vorrichtung nach Anspruch 3, wobei die Wärmequelle (400) der bewegungslosen Fluid-Pumpeinrichtung (200) auf dem Joule-Effekt und/oder Sonnenenergie basiert.

5. Tragbare elektronische Vorrichtung nach einem der Ansprüche 1 bis 4, wobei die Fluid-Messeinrichtung (300) für wenigstens eine Art von Molekül empfindlich ist.

6. Tragbare elektronische Vorrichtung nach einem der Ansprüche 1 bis 5, wobei die Fluid-Messeinrichtung (300) einen Metalloxid(MOX)-Sensor und/oder einen Kohlenstoff-Nanotube(CNT)-Sensor umfasst.

7. Tragbare elektronische Vorrichtung nach einem der Ansprüche 1 bis 6, die des Weiteren eine auf umgekehrter thermischer Transpiration basierende Fluid-Pumpeinrichtung (205) umfasst, die zum Ausstoßen des Probenentnahme-Volumens (47, 49) ausgeführt ist.

8. Tragbare elektronische Vorrichtung nach einem der Ansprüche 1 bis 7, wobei die bewegungslose Fluid-Pumpeinrichtung (200) eine reversible bewegungslose Fluid-Pumpeinrichtung (207) umfasst, die thermische Transpiration nutzt, um das Probenentnahme-Volumen (51, 53) mit einem bidirektionalen Strom zu bewegen.

## Revendications

1. Dispositif de télécommunication portable comprenant au moins un dispositif de détection de fluide (100, 103, 105) ;
le dispositif de détection de fluide (100, 103, 105) comprenant :
un moyen de détection de fluide (300) configuré pour analyser un volume d'échantillonnage (41, 45, 53) ; et
un moyen de déplacement de fluide comprenant un moyen de pompage de fluide sans mouvement (200) recourant à une transpiration thermique pour déplacer le volume d'échantillonnage (41, 45, 47, 49, 51, 53) ;
dans lequel le dispositif de détection de fluide (100, 103, 105) est positionné au sein de la même ouverture (13) du boîtier (2) du dispositif portable (1) qu'au moins une autre unité fonctionnelle (107) et plus avant à l'intérieur dudit boîtier (2) que l'au moins une autre unité fonctionnelle (107).

2. Le dispositif électronique portable selon la revendication 1, dans lequel le fluide est un gaz.

3. Le dispositif électronique portable selon la revendication 1 ou 2, dans lequel le moyen de pompage de fluide sans mouvement (200) est alimenté par une source de chaleur (400).

4. Le dispositif électronique portable selon la revendication 3, dans lequel la source de chaleur (400) du moyen de pompage de fluide sans mouvement (200) repose sur un effet Joule et/ou une énergie solaire.

5. Le dispositif électronique portable selon l'une des revendications 1 à 4, dans lequel le moyen de détection de fluide (300) est sensible à au moins un type de molécule.

6. Le dispositif électronique portable selon l'une des revendications 1 à 5, dans lequel le moyen de détection de fluide (300) comprend un capteur à oxyde métallique (MOX) et/ou un nanotube de carbone (CNT).

7. Le dispositif électronique portable selon l'une des revendications 1 à 6, comprenant en outre un moyen de pompage de fluide fondé sur une transpiration thermique inversée (205) configuré pour éjecter le volume d'échantillonnage (47, 49).

8. Le dispositif électronique portable selon une des revendications 1 à 7, dans lequel le moyen de pompage de fluide sans mouvement (200) comprend un moyen de pompage de fluide sans mouvement réversible (207) utilisant une transpiration thermique pour déplacer le volume d'échantillonnage (51, 53) avec un flux bidirectionnel.
